# EUROPEAN PATENT APPLICATION

(11) **EP 1 624 428 A1**
(43) Date of publication of application: **08.02.2006**
(21) Application number: 04732498.3
(22) Date of filing: 12.05.2004
(51) Int. Cl.: G08C 17/02, A61B 5/00

(54) **DEVICE FOR TRANSMITTING DATA VIA THE INTERNET**

(30) Priority: 14.05.2003 JP 2003136159
(71) Applicant: JMS Co. Ltd., Hiroshima-shi, Hiroshima 730-8652 (JP); Soho Corporation, Ltd., Tokyo 101-0021 (JP); Nakamoto, Hidetomo, Tokyo 179-0085 (JP)
(72) Inventor: NAKAMOTO, Hidetomo, Nerima-ku, Tokyo 179-0085 (JP); YOSHIMOTO, Mitsuo, Hiroshima-shi, Hiroshima 730-8652 (JP); KASHIHARA, Tomio, Sotokanda, Chiyoda-ku, Tokyo 101-0021 (JP); RYUZAKI, Munekazu, Tokyo 152-0032 (JP); SONE, Masayoshi, Shibuya-ku, Tokyo 150-0001 (JP); NISHIDA, Eiichi, Kitakyushu-shi, Fukuoka 805-0016 (JP)
(74) Representative: Grape & Schwarzensteiner
(86) International application number: PCT/JP2004/006718
(87) International publication number: WO 2004/104964

(57) **Abstract**

A serial terminal (9) for a serial connection to a target device for data acquisition, a data transfer terminal (12) to be connected to a mobile phone (5) so as to allow a data transfer, a data acquisition portion (13) for acquiring device data from the target device for data acquisition by a serial communication via the serial terminal, a data transfer portion (14) for adding transmission instruction data to the device data so as to create transfer data and transferring the transfer data via the data transfer terminal, and a control portion (15) for executing an operation of acquiring the device data by the data acquisition portion and an operation of transferring the transfer data by the data transfer portion are provided, and the transmission instruction data correspond to an instruction to cause the mobile phone to conduct an operation of transmitting the device data to a predetermined server via the internet. It becomes possible to transfer blood pressure data or the like from a remote location via the internet in an easy manner.

## Description

### Technical Field

The present invention relates to a transfer apparatus for transferring device data acquired from, for example, a sphygmomanometer to a server connected to the internet in a simple manner, and a method for transferring device data using the same.

### Background Art

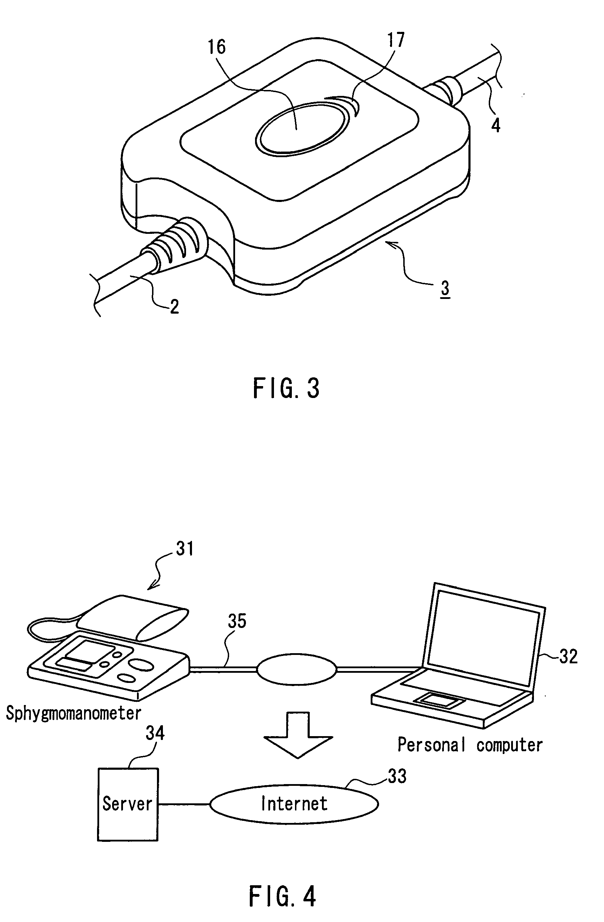

FIG. 4 schematically shows a configuration of a conventional device data collection system. This data collection system includes a sphygmomanometer 31 for measuring a blood pressure of a patient, a personal computer 32 and a server 34 that is connected to the internet 33 and for accumulating blood pressure data.

The sphygmomanometer 31 is connected to the personal computer 32 via a serial cable 35. The personal computer 32 is connectable to the server 34 via the internet 33. With this data collection system, it is possible to accumulate blood pressure data indicating a patient's blood pressure measured by the sphygmomanometer 31 in the server 34.

In the data collection system having the above-described configuration, when the blood pressure of a patient is measured with the sphygmomanometer 31, the blood pressure data indicating the measured blood pressure are transferred via the serial cable 35 to the personal computer 32. The blood pressure data transferred to the personal computer 32 are accumulated in the server 34 via the internet 33.

With this data collection system, it becomes easier to collect the blood pressure data of a patient who lives in a remote location, thus allowing a doctor to diagnose a health condition relating to the blood pressure of the patient who lives in a remote location (for example, see JP 2002-355305 A).

However, with such a configuration of the data collection system, in order to transfer the patient's blood pressure data measured with the sphygmomanometer 31 to the server 34, it is necessary to operate the personal computer 32 and connect it to the internet 33. The personal computer 32 has a long starting time from turning on a power until an operable state is achieved. Also, for operating a software for transferring the blood pressure data to the server 34, the operations of input instruments such as a mouse and a keyboard are complicated.

In particular, this is a serious problem if the patient is an elderly person or is not accustomed to operating information instruments such as a personal computer. In many cases, the patient whose blood pressure is measured with the sphygmomanometer 31 is an elderly person who is not accustomed to operating a personal computer or the like.

As a result, there has been a problem that it is difficult for the patient who lives in a remote location to transfer his/ her own blood pressure data to the server 34 via the internet 33.

### Disclosure of Invention

It is an object of the present invention to provide a data transfer apparatus making it possible to transfer device data such as blood pressure data acquired from a device such as a sphygmomanometer provided in a remote location to a server connected to the internet in an easy manner.

A data transfer apparatus according to the present invention includes a serial terminal for a serial connection to a target device for data acquisition, a data transfer terminal to be connected to a mobile phone so as to allow a data transfer, a data acquisition portion for acquiring device data from the target device for data acquisition by a serial communication via the serial terminal, a data transfer portion for adding transmission instruction data to the device data so as to create transfer data and transferring the transfer data via the data transfer terminal, and a control portion for executing an operation of acquiring the device data by the data acquisition portion and an operation of transferring the transfer data by the data transfer portion. The transmission instruction data correspond to an instruction to cause the mobile phone to conduct an operation of transmitting the device data to a predetermined server via the internet.

### Brief Description of Drawings

FIG. 1 is a block diagram schematically showing a configuration of a data collection system using data transfer apparatuses in an embodiment of the present invention.
FIG. 2 is a block diagram showing the configuration of the above-mentioned data transfer apparatus.
FIG. 3 is a perspective view showing an external appearance of the above-mentioned data transfer apparatus.
FIG. 4 schematically shows a configuration of a conventional data collection system.

### Description of the Invention

With the data transfer apparatus according to the present invention, device data acquired from a device such as a sphygmomanometer can be transferred from a mobile phone via the internet to a predetermined server by a simple operation.

The data transfer apparatus according to the present invention preferably is constituted so that an activation switch is provided, and by an operation of the activation switch, the control portion starts the operation by the data acquisition portion and the operation by the data transfer portion.

Also, it is preferable further to include a memory storing a serial number for distinguishing the data transfer apparatus from one another, and that the data transfer portion adds the serial number to the device data so as to create data to be transmitted to the server.

Further, the data transfer portion preferably has a configuration including an emulator for converting data into an emulation code according to a keyboard emulation format for operating the mobile phone, and converting the transfer data into the emulation code and transferring the emulation code.

In this configuration, it is preferable that the data transfer portion has a data creation portion and a transfer processing portion, the data creation portion creates data containing the device data and the transmission instruction data and supplies them to the transfer processing portion, and the transfer processing portion converts the data supplied from the data creation portion into the emulation code and outputs the emulation code from the data transfer terminal.

Furthermore, in the data transfer apparatus according to the present invention, it is preferable that the control portion has a function of detecting whether connection states with respect to the target device for data acquisition and the mobile phone are good or bad, and a display portion is provided for indicating whether the connection states are good or bad by a blinking state controlled by the control portion.

In the data transfer apparatus according to the present invention, the target device for data acquisition can be a medical instrument, an electric meter for measuring an electricity consumption amount, a water meter for measuring a running water consumption amount, production equipment capable of outputting log data or an electric home appliance capable of outputting data indicating an operation state. The medical instrument can be a sphygmomanometer, a pulsimeter or a blood sugar level meter.

A method for transferring device data according to the present invention is a method using the data transfer apparatus having any of the configurations described above so as to transfer the device data acquired from the target device for data acquisition from the mobile phone via the internet. The method is executed by a procedure including turning on a power source of the mobile phone, turning on a power source of the data transfer apparatus and a power source of the target device for data acquisition, connecting the data transfer apparatus to the target device for data acquisition by a serial cable, and connecting the data transfer apparatus to the mobile phone by a transfer cable, in any order, followed by activating the control portion of the data transfer apparatus.

The following is a specific description of an embodiment of the present invention, with reference to the accompanying drawings.

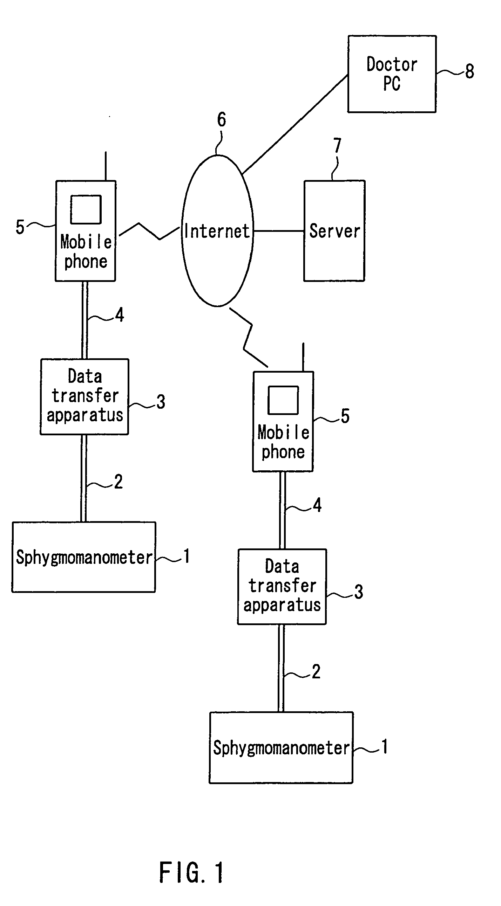

FIG. 1 schematically shows a configuration of a data collection system using data transfer apparatuses in the present embodiment. This data collection system is for collecting blood pressure data obtained by measuring the blood pressure of individual patients from a plurality of sphygmomanometers 1. Each of the sphygmomanometers 1 is connected to a data transfer apparatus 3 via a serial cable 2. Each of the data transfer apparatuses 3 is connected to a mobile phone 5 via a transfer cable 4. Each of the mobile phones 5 is connectable to the internet 6.

The data collection system further includes a server 7 connected to the internet 6 for accumulating blood pressure data measured with each of the sphygmomanometers 1 and a personal computer for a doctor (in the following, referred to as a doctor PC) 8 that is connectable to the internet 6. The doctor PC 8 is provided for allowing a doctor to analyze the blood pressure data accumulated in the server 7.

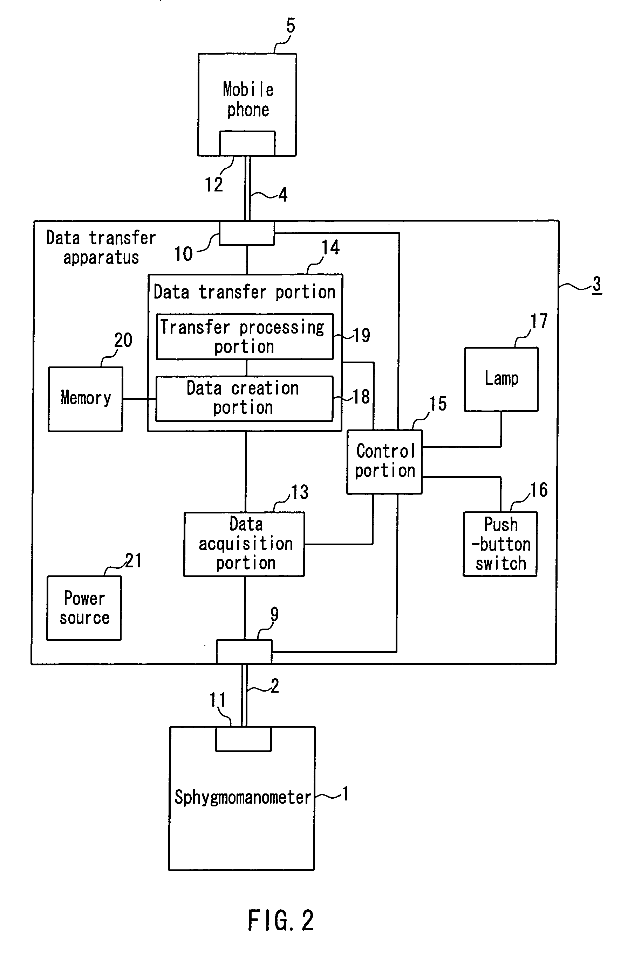

FIG. 2 is a block diagram showing a configuration of the data transfer apparatus 3 and a connection structure with the sphygmomanometer 1 and the mobile phone 5. The data transfer apparatus 3 includes a serial terminal 9 connected to one end of the serial cable 2 and a transfer terminal 10 connected to one end of the transfer cable 4. The other end of the serial cable 2 is connected to a serial terminal 11 provided in the sphygmomanometer 1. The other end of the transfer cable 4 is connected to a terminal 12 in the mobile phone 5. Respective cables and terminals can be connected or disconnected freely.

The data transfer apparatus 3 has a data acquisition portion 13, a data transfer portion 14 and a control portion 15 as major elements. These elements can be configured with a microprocessor. The data acquisition portion 13 is supplied with an output of the sphygmomanometer 1 via the serial terminal 11, the serial cable 2 and the serial terminal 9. An output of the data acquisition portion 13 is supplied to the data transfer portion 14. An output of the data transfer portion 14 is supplied to the mobile phone 5 via the transfer terminal 10, the transfer cable 4 and the transfer terminal 12. The control portion 15 controls operations of the data acquisition portion 13 and the data transfer portion 14.

A push-button switch 6 is connected to the control portion 15, and by an operation thereof, the control portion 15 exerts control so as to start a series of operations performed by the data acquisition portion 13 and the data transfer portion 14. The control portion 15 is connected to the serial terminal 9 and the transfer terminal 10 and determines whether a connection state of the serial cable 2 and that of the transfer cable 4 are good or bad. Since the determination method can be any usual methods, the specific description thereof will be omitted. The result of the determination is indicated by a lamp 17.

The data acquisition portion 13 is activated by the control of the control portion 15, automatically acquires the measured blood pressure data and sphygmomanometer data containing data specifying the date and time of measuring the blood pressure from the sphygmomanometer 1 and supplies them to the data transfer portion 14.

The data transfer portion 14 includes a data creation portion 18 and a transfer processing portion 19. The data transfer portion 14 is connected to a memory 20, and the memory 20 stores a serial number for identifying the individual data transfer apparatus 3 and transmission instruction data. The data creation portion 18 creates transfer data by adding the serial number and the transmission instruction data obtained from the memory 20 to the sphygmomanometer data obtained from the data acquisition portion 13, and supplies it to the transfer processing portion 19. The transfer processing portion 19 converts the transfer data into an emulation code representing a state in which a key or the like of the mobile phone 5 is pressed and transfers it to the mobile phone 5 according to a keyboard emulation format.

Based on the transmission instruction data contained in the transferred data, the mobile phone 5 carries out a processing for connecting to the internet 6 and a processing for transmitting the sphygmomanometer data and the serial number to the server 7. As a result, the sphygmomanometer data and the serial number are transferred from the mobile phone 5 via the internet 6 to the server 7.

Transfer processing from the mobile phone 5 is carried out based on a POST/GET method of the internet, for example. Thus, the transmission instruction data stored in the memory 20 of the data transfer apparatus 3 contain data regarding a menu selection of the mobile phone 5, an initiation of a function of the mobile phone 5 for connection to the internet and the connection, and a input of URL of the server to which the data are to be transferred. The transfer processing portion 19 converts these transmission instruction data and numerical values of the sphygmomanometer data and the serial number into an emulation code and outputs this emulation code. It should be noted that, in the case where the mobile phone 5 has an emulator, the conversion processing by the transfer processing portion 19 is not necessary.

A power source 21 provided in the data transfer apparatus 3 is constituted by, for example, a dry battery and supplies a voltage for operating the data acquisition portion 13, the data transfer portion 14, the control portion 15, the lamp 17 and the memory 20.

FIG. 3 is a perspective view showing an external appearance of the data transfer apparatus 3 according to the present embodiment. The data transfer apparatus 3 has a substantially rectangular shape, and the push-button switch 16 having an elliptical shape is provided at the center of an upper surface of the data transfer apparatus 3. The lamp 17 having a crescent shape is provided adjacent to the push-button switch 16.

The operation of the data collection system configured as above will be described. When the power source of the data transfer apparatus 3 is turned on, the control portion 15 detects whether the connection states of the serial cable 2 and the transfer cable 4 are good or bad. If the connection state is good, the lamp 17 is made to light up. If the connection state of the serial cable 2 or the transfer cable 4 is bad, the lamp 17 is made to blink. In this way, it is possible to adjust the connection state of the serial cable 2 or the transfer cable 4, thereby preventing a transfer error of the sphygmomanometer data caused by a poor connection.

Next, when a patient pushes down the push-button switch 16, the data acquisition portion 13 acquires the sphygmomanometer data measured with the sphygmomanometer 1 by serial communication. The acquired sphygmomanometer data are supplied to the data transfer portion 14.

Subsequently, the data transfer portion 14 first causes the data creation portion 18 to add the serial number and the transmission instruction data stored in the memory 20 to the sphygmomanometer data and then supplies them to the transfer processing portion 19. The transfer processing portion 19 converts the data according to the keyboard emulation format of the mobile phone 5 and transfers them to the mobile phone 5.

The mobile phone 5 carries out an operation for connection to the internet 6 according to the transmission instruction data in the transfer data received from the data transfer portion 14 and an operation of transmitting the sphygmomanometer data and the serial number via the internet 6 to the server 7. Then, the server 7 receives and accumulates the sphygmomanometer data and the serial number transmitted from each mobile phone 5.

As a result, it becomes possible to acquire the sphygmomanometer data accumulated in the server 7 via the internet 6 and analyze them with the doctor PC 8.

As described above, with the data collection system according to the present embodiment, the data transfer apparatus 3 substantially automatically transfers the sphygmomanometer data measured with the sphygmomanometer 1 from the mobile phone 5 via the internet 6 to the server 7. Thus, the sphygmomanometer data can be transmitted in a simple manner without using a personal computer whose operation is complicated. In other words, the operation of the data transfer apparatus 3 only involves the connections of the serial cable 2 and the transfer cable 4 and a single push of the push-button switch 16 and, thus, is extremely easy.

Further, since the data transfer apparatus 3 is operated by the power source 21 constituted by a dry battery, it is easy to handle. The mobile phone to be connected to the data transfer apparatus 3 is widespread and easily available and, because of its small size, easy to handle. Accordingly, for example, it is easy for a patient in a remote location to measure his/ her own blood pressure with the sphygmomanometer 1 three or four times a day everyday and transmit the sphygmomanometer data to the server 7 every time the blood pressure is measured. Alternatively, it also is easy to transmit the sphygmomanometer data for one day at one time to the server 7.

The following is an example of an operation procedure at the time of transmission. First, the power source of the mobile phone 5 is turned on, and then the power source of the data transfer apparatus 3 and that of the sphygmomanometer 1 are turned on. Next, the data transfer apparatus 3 and the mobile phone 5 are connected by the transfer cable 4, and the data transfer apparatus 3 and the sphygmomanometer 1 are connected by the serial cable 2. Thereafter, when the push-button switch 16 of the data transfer apparatus 3 is pushed down, the blood pressure data that have been measured and accumulated in the sphygmomanometer 1 by then are transmitted automatically from the sphygmomanometer 1 via the mobile phone 5 and the internet 6 to the server 7.

Further, since the sphygmomanometer data and the serial number transmitted from the mobile phone 5 via the internet 6 to the server 7 consist of a string of numerals, it is not possible to determine the ID of a patient even if these data are intercepted on the internet 6. Consequently, the privacy of the patient can be protected.

A doctor can specify the patient only after he/ she matches the serial number transferred to the server and a patient ID. The above-noted serial number is given to each data transfer apparatus 3, and thus, no means for identifying a sphygmomanometer or a mobile phone is needed. For example, it is not necessary to input an ID to the sphygmomanometer or the mobile phone, so that the operation is simple. Also, since one data transfer apparatus can be made to correspond to one patient, the sphygmomanometer and the mobile phone can be shared among a plurality of patients. Furthermore, a doctor instantly can analyze the blood pressure data collected in the server 7 using the doctor PC 8, so that any abnormal values in the blood pressure data can be recognized in a timely manner. Consequently, the doctor can start a treatment on the patient immediately.

Although the above-described embodiment has been directed to an example in which the data transfer apparatus 3 is connected to the sphygmomanometer 1, the present invention is not limited to this. In other words, any electric appliances can be connected to the data transfer apparatus 3 according to the present embodiment so as to obtain a similar effect as long as it is capable of transferring data to be handled by serial communication. For example, the data transfer apparatus may be connected to other medical instruments such as a pulsimeter for measuring a patient's pulsation, a dialysis unit and a blood sugar level meter.

Moreover, the data transfer apparatus 3 may be connected to an electric meter for measuring an electricity consumption amount, a water meter for measuring a running water consumption amount or the like. Alternatively, the data transfer apparatus 3 can be connected to production equipment for producing articles, and log data associated with an operation state of the production equipment can be transferred. Alternatively, the data transfer apparatus 3 can be connected to an electric home appliance, and data indicating an operation state of the electric home appliance, for example, data indicating whether or not a battery is exhausted can be transferred.

### Industrial Applicability

In accordance with the present invention, it is possible to provide a data transfer apparatus making it possible to transfer device data such as sphygmomanometer data acquired from a device such as a sphygmomanometer provided in a remote location to a server connected to the internet in an easy manner.

## Claims

1. A data transfer apparatus comprising:
a serial terminal for a serial connection to a target device for data acquisition;
a data transfer terminal to be connected to a mobile phone so as to allow a data transfer;
a data acquisition portion for acquiring device data from the target device for data acquisition by a serial communication via the serial terminal;
a data transfer portion for adding transmission instruction data to the device data so as to create transfer data and transferring the transfer data via the data transfer terminal; and
a control portion for executing an operation of acquiring the device data by the data acquisition portion and an operation of transferring the transfer data by the data transfer portion;
wherein the transmission instruction data correspond to an instruction to cause the mobile phone to conduct an operation of transmitting the device data to a predetermined server via the internet.

2. The data transfer apparatus according to claim 1, wherein an activation switch is provided, and by an operation of the activation switch, the control portion starts the operation by the data acquisition portion and the operation by the data transfer portion.

3. The data transfer apparatus according to claim 1 or 2, further comprising a memory storing a serial number for distinguishing the data transfer apparatus from one another, wherein the data transfer portion adds the serial number to the device data so as to create data to be transmitted to the server.

4. The data transfer apparatus according to any one of claims 1 to 3, wherein the data transfer portion includes an emulator for converting data into an emulation code according to a keyboard emulation format for operating the mobile phone, and converts the transfer data into the emulation code and transfers the emulation code.

5. The data transfer apparatus according to claim 4, wherein the data transfer portion has a data creation portion and a transfer processing portion, the data creation portion creates data containing the device data and the transmission instruction data and supplies them to the transfer processing portion, and the transfer processing portion converts the data supplied from the data creation portion into the emulation code and outputs the emulation code from the data transfer terminal.

6. The data transfer apparatus according to claim 1, wherein the control portion has a function of detecting whether connection states with respect to the target device for data acquisition and the mobile phone are good or bad, and a display portion is provided for indicating whether the connection states are good or bad by a blinking state controlled by the control portion.

7. The data transfer apparatus according to claim 1, wherein the target device for data acquisition is a medical instrument, an electric meter for measuring an electricity consumption amount, a water meter for measuring a running water consumption amount, production equipment capable of outputting log data or an electric home appliance capable of outputting data indicating an operation state.

8. The data transfer apparatus according to claim 7, wherein the medical instrument is a sphygmomanometer, a pulsimeter or a blood sugar level meter.

9. A method for transferring device data using the data transfer apparatus according to any of claims 1 to 8 so as to transfer the device data acquired from the target device for data acquisition from the mobile phone via the internet, the method comprising:
turning on a power source of the mobile phone;
turning on a power source of the data transfer apparatus and a power source of the target device for data acquisition;
connecting the data transfer apparatus to the target device for data acquisition by a serial cable; and
connecting the data transfer apparatus to the mobile phone by a transfer cable; in any order, followed by
activating the control portion of the data transfer apparatus.
